# EUROPÄISCHE PATENTSCHRIFT

(11) **EP 3 622 908 B1**
(45) Veröffentlichungstag und Bekanntmachung des Hinweises auf die Patenterteilung: **06.04.2022**
(21) Anmeldenummer: 19187258.9
(22) Anmeldetag: 19.07.2019
(51) Int. Cl.: A61B 17/34, A61B 90/13

(54) **AUSRICHTELEMENT ZUM AUSRICHTEN EINER NADELFÜHRUNG; AUSRICHTANORDNUNG; FÜHRUNGSANORDNUNG; BEHANDLUNGSANORDNUNG SOWIE VERFAHREN**
ALIGNMENT ELEMENT FOR ALIGNING A NEEDLE GUIDE; ALIGNMENT ARRANGEMENT; GUIDING ARRANGEMENT; TREATMENT ARRANGEMENT AND METHOD
ÉLÉMENT D'ALIGNEMENT PERMETTANT D'ALIGNER UN GUIDE D'AIGUILLE, DISPOSITIF D'ALIGNEMENT; DISPOSITIF DE GUIDAGE, DISPOSITIF DE TRAITEMENT AINSI QUE PROCÉDÉ

(30) Priorität: 13.09.2018 DE 102018215599
(43) Veröffentlichungstag der Anmeldung: 18.03.2020
(73) Patentinhaber: Siemens Healthcare GmbH, 91052 Erlangen (DE)
(72) Erfinder: Regensburger, Alois, 91058 Erlangen (DE); Alzaga, Amilcar, 90453 Nürnberg (DE)

(56) Entgegenhaltungen:
- WO-A1-93/15683
- WO-A1-95/21582
- US-A1- 2001 053 915
- US-A1- 2007 055 291
- US-A1- 2008 146 963
- US-A1- 2017 056 062

## Beschreibung

Die Erfindung betrifft ein Ausrichtelement zum Ausrichten einer Nadelführung, welche zur Längsführung einer medizinischen Nadel eingerichtet ist. Zur Erfindung gehört außerdem eine Ausrichtanordnung mit einem solchen Ausrichtelement.

Weitere Aspekte der Erfindung betreffen eine Führungsanordnung und eine Behandlungsanordnung jeweils zur Längsführung einer medizinischen Nadel. Außerdem ist Teil der Erfindung ein Verfahren zum Ausrichten einer Nadelführung.

In vielen Fällen ist es für medizinische Behandlungen nötig, eine medizinische Nadel zielgerichtet, das bedeutet entlang eines genau festgelegten Nadelpfades, in ein Objekt, insbesondere einen Patienten, einzubringen. Insbesondere bei der Behandlung im Körperinneren des Pateinten, beispielsweise innere Organe, ist die exakte Führung der Nadel entlang des Nadelpfades besonders wichtig, um einerseits den korrekten Pfad zwischen Gewebe und Knochen hindurch und andererseits die gezielte Behandlung des jeweiligen Organs zu gewährleisten. In diesem Kontext ist beispielsweise das Überwachen eines Einstechvorgangs der Nadel mittels Fluoroskopie möglich. Nachteilig hierbei ist die hohe Belastung mit Röntgenstrahlen. Aus der US 9 095 361 B2 ist ein alternatives Verfahren zum Führen einer Nadel zu entnehmen. Hierbei wird die Nadel im Licht einer Lichtquelle in einen gewünschten Nadeleinstichwinkel gebracht, wobei an der Nadel eine Kappe angeordnet ist, durch welche ein Laser oder anderes Licht zur Zielführung hindurch scheinen kann.

Dokument US 2007/055291 A1 beschreibt ein Bildgebungssystem mit einer Fluoroskopieeinheit, die eine Lasereinheit zur Erzeugung eines Lichtstrahls zur Markierung einer Trajektorie für eine Kanüle, die als Nadel ausgestaltet sein kann, nachdem die Fluoroskopieeinheit die Trajektorie identifiziert hat, aufweist. Der Lichtstrahl verläuft durch die Kanüle, wenn diese bezüglich der Trajektorie ausgerichtet ist. Ein Orientierungsmarker kann in die Kanüle eingeführt werden, um die Ausrichtung des Lichtstrahls zu erleichtern.

Es ist Aufgabe der vorliegenden Erfindung, eine verbesserte Führung für eine medizinische Nadel zu ermöglichen.

Diese Aufgabe wird erfindungsgemäß gelöst durch die unabhängigen Patentansprüche. Vorteilhafte Ausführungsformen mit zweckmäßigen Weiterbildungen sind Gegenstand der Unteransprüche.

Ein erster Aspekt der Erfindung betrifft ein Ausrichtelement zum Ausrichten einer Nadelführung, welche zur Längsführung einer medizinischen Nadel eingerichtet ist, mit
- einem Verbindungselement zum Anordnen des Ausrichtelements an einer solchen Nadelführung, und
- einer Lichtführungseinrichtung für eine vorbestimmte Lichtverteilung, wobei die Lichtführungseinrichtung nur bei einer durch deren Geometrie vorgegebenen Pose relativ zu der Lichtverteilung ein vorbestimmtes Lichtmuster erzeugt.

Bei der Pose handelt es sich um die Kombination von Position und Orientierung im dreidimensionalen Raum. Dabei kann die vorgegebene Pose bezüglich fünf ihrer sechs Freiheitsgrade festgelegt sein. Als einziger Freiheitsgrad kann eine Verschiebung entlang der Längsführung frei wählbar sein. Mit anderen Worten ist die vorgegebene Pose, in welcher die Lichtführungseinrichtung das vorbestimmte Lichtmuster erzeugt, bezüglich zweier translatorischer Freiheitsgrade und dreier Raumwinkel durch die Geometrie der Lichtführungseinrichtung festgelegt.

Zur Längsführung der medizinischen Nadel kann durch die Nadelführung eine Bewegung der medizinischen Nadel zumindest entlang einer Raumrichtung, vorzugsweise jedoch entlang zweier Raumrichtungen, zumindest teilweise eingeschränkt werden. Eine Bewegung der Nadel kann somit in alle Raumrichtungen quer zu einer Längsausdehnung der Längsführung eingeschränkt oder unterbunden werden. Durch die Längsführung kann sichergestellt sein, dass eine Bewegung der Nadel nur parallel zu dem zuvor bestimmten Nadelpfad möglich ist, wenn die Nadelführung entsprechend dem zuvor bestimmten Nadelpfad ausgerichtet ist.

Das Verbindungselement ist zum Herstellen einer mechanischen Verbindung zwischen dem Ausrichtelement und der Nadelführung ausgebildet. Insbesondere ist da Verbindungselement dazu ausgebildet, das Ausrichtelement in einer vorbestimmten Relativposition beziehungsweise einer vorbestimmten Pose relativ zur Nadelführung an der Nadelführung anzuordnen. Das Anordnen kann temporär oder dauerhaft sein. Mit anderen Worten kann das Verbindungselement zum abwechselnden Anordnen und Abnehmen des Ausrichtelements an der Nadelführung oder zur festen, das heißt nicht ohne weiteres trennbaren, Verbindung des Ausrichtelements mit der Nadelführung ausgebildet sein.

Bei der vorbestimmten Lichtverteilung handelt es sich vorteilhafterweise um eine übliche Lichtverteilung einer üblichen Lichtquelle zum Darstellen eines Nadelpfades. Insbesondere handelt es sich bei der vorbestimmten Lichtverteilung um eine vorbestimmte Verteilung von Laserlicht. Beispielsweise ist die vorbestimmte Lichtverteilung derart ausgeführt, dass durch diese zwei überkreuzende Linien auf eine Einstichstelle, in welche die medizinische Nadel in ein Körperteil eingeführt werden soll, projiziert werden. Das Ausrichtelement kann demnach dazu ausgestaltet sein, mit einer derartigen Projektionseinrichtung, welche die genannte Lichtquelle umfasst, auf bestimmungsgemäße Weise verwendet zu werden. Die Lichtführungseinrichtung ist dazu ausgebildet, das vorbestimmte Lichtmuster nur zu erzeugen, wenn die vorbestimmte Lichtverteilung aus einem durch die vorgegebene Pose bestimmten Raumwinkel auf die Lichtführungseinrichtung trifft. Die vorbestimmte Lichtverteilung trifft insbesondere dann in einem durch die Geometrie der Lichtführungseinrichtung vorgegebenen Raumwinkel auf die Lichtführungseinrichtung, wenn sich die Lichtführungseinrichtung in der vorgegebenen Pose relativ zu der Lichtverteilung befindet. Das vorbestimmte Lichtmuster kann durch geeignetes Reflektieren und/oder Absorption zumindest eines Teils der Lichtverteilung durch die Lichtführungseinrichtung erfolgen.

Durch die vorbestimmte Lichtverteilung ist bei einer bestimmungsgemäßen Anwendung des Ausrichtelements insbesondere ein zuvor bestimmter Nadelpfad, entlang welchem die medizinische Nadel zu führen ist, dargestellt beziehungsweise projiziert. Insbesondere werden durch die beiden Linien, welche auf die Einstichstelle projiziert werden, zwei Ebenen aufgespannt. Der Nadelpfad kann hierbei einer Schnittgeraden der beiden aufgespannten Ebenen entsprechen.

Insgesamt ist durch das Ausrichtelement gewährleistet, dass eine verbesserte Ausrichtung der Nadelführung ermöglicht wird. Aus der verbesserten Ausrichtung der Nadelführung ergibt sich eine besonders genaue Führung der medizinischen Nadel entlang des zuvor bestimmten Nadelpfades. Durch die Nutzung einer solchen Nadelführung ist es insbesondere möglich, Behandlungsfehler beim Einführen der Nadel durch einen Arzt, beispielsweise aufgrund zittriger Hände, zu vermeiden beziehungsweise wenigstens die Wahrscheinlichkeit hierfür zu verringern.

Es ist vorgesehen, dass die Lichtführungseinrichtung zumindest zwei Erkennungsmarken auf zwei unterschiedlichen, insbesondere parallelen, Ebenen aufweist und die Erkennungsmarken nur im Falle der vorgegebenen Pose des Ausrichtelements durch die vorbestimmte Lichtverteilung gleichzeitig beleuchtet sind. Mit anderen Worten weist die Lichtführungseinrichtung eine Vielzahl, das bedeutet zumindest zwei, vorteilhafterweise jedoch zumindest zwei, Erkennungsmarken auf, welche nur dann gleichzeitig durch die vorbestimmte Lichtverteilung beleuchtet sind, wenn sich die Lichtführungseinrichtung in der vorbestimmten Pose relativ zu der Lichtverteilung befindet.

Diese Vielzahl an Erkennungsmarken ist insbesondere auch auf den zwei unterschiedlichen, insbesondere parallelen, Ebenen angeordnet. Vorteilhafterweise sind in jeder der beiden unterschiedlichen Ebenen jeweils zumindest zwei Erkennungsmarken angeordnet. In besonders vorteilhafter Ausgestaltung sind die zumindest zwei Erkennungsmarken einer Ebene nicht parallel zueinander ausgerichtet. Insbesondere können die Ebenen vollständig oder teilweise senkrecht zu dem als Stift ausgebildeten Verbindungselement sein. Durch die Aufteilung ergibt sich eine besonders gute Erkennbarkeit des vorbestimmten Lichtmusters. Insbesondere besteht das vorbestimmte Lichtmuster darin, dass die Erkennungsmarken gleichzeitig durch das vorbestimmte Lichtmuster beleuchtet sind.

Gemäß einer Weiterbildung ist vorgesehen, dass die Lichtführungseinrichtung ein erstes und ein zweites scheibenförmiges Gebilde aufweist, wobei das erste und das zweite scheibenförmige Gebilde jeweils in einer unterschiedlichen der Ebenen angeordnet sind, und wobei Erkennungsmarken der ersten Ebene als Spalte und Erkennungsmarken der zweiten Ebene als hierzu korrespondierende Markierungen ausgeführt sind. Mit anderen Worten sind die erste und die zweite Ebene in den oben genannten zwei unterschiedlichen, insbesondere parallelen, Ebenen angeordnet. Dementsprechend können die scheibenförmigen Gebilde ebenfalls parallel zueinander sein. Das vorbestimmte Lichtmuster kann darin bestehen, dass die Lichtverteilung durch die Spalten der ersten Ebene hindurchtritt und durch die Spalte hindurch auf die Markierungen der zweiten Ebenen trifft. Insbesondere ist eine zu einem Spalt korrespondierende Markierung bezüglich einer Führungsrichtung der Längsführung (also der Richtung, entlang welcher die medizinische Nadel durch die Längsführung geführt wird) unterhalb des jeweiligen Spalts angeordnet. Auf diese Weise ergibt sich eine besonders einfache und effiziente Geometrie für die Lichtführungseinrichtung.

Gemäß einer Weiterbildung ist vorgesehen, dass das Verbindungselement zumindest partiell als Stift ausgeführt ist. Der Stift kann dabei ähnlich oder zumindest partiell entsprechend der medizinischen Nadel ausgeführt sein. Auf diese Weise ist sichergestellt, dass das Ausrichtelement mittels des Verbindungselements auf analoge Art und Weise wie die medizinische Nadel in die Nadelführung eingeführt werden kann beziehungsweise an der Nadelführung angeordnet werden kann. In der bestimmungsgemäßen Position der Nadelführung, in welcher die medizinische Nadel entlang dem zuvor bestimmten Nadelpfad geführt werden kann, kann der partiell als Stift ausgeführte Teil des Verbindungselements parallel zu dem Nadelpfad sein.

Ein zweiter Aspekt der Erfindung betrifft eine Ausrichtanordnung, welche zur Längsführung einer medizinischen Nadel eingerichtet ist, mit
- einer ausrichtbaren Nadelführung, und
- dem im Vorherigen beschriebenen Ausrichtelement. Auch die Nadelführung wurde im Vorherigen bereits beschrieben. Aus diesem Grund gelten die zuvor getätigten Aussagen und offenbarten Merkmale auch für die Nadelführung, welche Teil der Ausrichtanordnung ist.

Gemäß einer Weiterbildung ist vorgesehen, dass die Nadelführung zur Aufnahme des Ausrichtelements und zur Führung der medizinischen Nadel dasselbe Mittel aufweist. Mit anderen Worten ist dasselbe Mittel der Nadelführung sowohl zur Aufnahme des Ausrichtelements als auch zur Führung der medizinischen Nadel ausgebildet. Mit anderen Worten kann das Ausrichtelement, insbesondere mit dessen Verbindungselement, welches vorteilhafterweise partiell als Stift ausgeführt ist, in das Mittel der Nadelführung eingeführt werden, welches auch zur Längsführung der medizinischen Nadel ausgebildet ist. Auf diese Weise kann zunächst die Nadelführung mit Hilfe des Ausrichtelements ausgerichtet werden und anschließend (nach Entfernung des Ausrichtelements) durch dasselbe Mittel die Nadel geführt werden. Insbesondere handelt es sich bei dem besagten Mittel um eine Bohrung.

Gemäß einer anderen Ausführungsform ist vorgesehen, dass die Nadelführung und das Ausrichtelement einstückig miteinander verbunden sind. In diesem Fall kann die Nadelführung fest mit dem Ausrichtelement verbunden sein. Bei dieser Ausführungsform ist insbesondere nicht vorgesehen, dass zum Führen der medizinischen Nadel das Ausrichtelement von der Nadelführung abgenommen beziehungsweise entfernt wird. Dadurch, dass das Ausrichtelement gegenüber einem durch die Nadelführung vorgegebenen Nadelpfad verschoben ist, muss bei dieser Ausführungsform berücksichtigt werden, dass die vorbestimmte Lichtverteilung auf gleichartige Weise verschoben wird. Vorteil dieser Ausführungsform ist, dass während des Führens der Nadel die korrekte Position der Nadelführung kontrolliert werden kann.

Gemäß einer Weiterbildung ist vorgesehen, dass zumindest eine der zwei unterschiedlichen Ebenen senkrecht zu einer Führungsrichtung der Nadelführung ausgerichtet ist. Insbesondere sind beide der zwei unterschiedlichen Ebenen senkrecht zu der Führungsrichtung ausgerichtet. Mit anderen Worten ist die Nadelführung dazu ausgebildet, die medizinische Nadel quer zu zumindest einer der zwei unterschiedlichen Ebenen zu führen. Bezug genommen wird hierbei auf die Ebenen, auf welchen die Erkennungsmarken des Ausrichtelements angeordnet sind. Die hier beschriebene Anordnung hat sich als besonders vorteilhaft erwiesen.

Gemäß einer Weiterbildung ist vorgesehen, dass sich eine Projektionen des ersten und des zweiten scheibenförmigen Gebildes in Richtung der Längsführung auf eine der Ebenen zumindest bereichsweise überschneiden. Mit anderen Worten liegen die beiden Ebenen bezüglich der Führungsrichtung der Nadelführung zumindest teilweise übereinander. Diese Ausgestaltungsform ermöglicht ein noch vorteilhafteres Ausrichten der Nadelführung.

Ein weiterer Aspekt der Erfindung betrifft eine Führungsanordnung zur Längsführung einer medizinischen Nadel, mit
- einer Ausrichtanordnung der oben genannten Art,
- einem Befestigungsteil zum Befestigen der Führungsanordnung an einer Patientenaufnahme zur Aufnahme eines Patienten, und
- einem Halteteil zum Verbinden der Nadelführung und des Befestigungsteils miteinander, wobei der Halteteil zumindest ein Verstellelement zum Verstellen einer Relativposition zwischen Befestigungsteil und Nadelführung aufweist.

Durch die Führungsanordnung wird dementsprechend eine Relativposition zwischen der Nadelführung und der Patientenaufnahme eingestellt. Der Befestigungsteil kann beispielsweise einen Schraubmechanismus, einen Klemmmechanismus oder einen beliebigen anderen Mechanismus aufweisen, um die Führungsanordnung an der Patientenaufnahme anzuordnen. Das Halteteil kann beispielsweise als Teleskoparm mit einem oder mehreren Auszieh- und/oder Klappelementen oder als verstellbares Schienensystem ausgeführt sein. Die Nadelführung kann über ein Gelenk, beispielsweise ein Kugelgelenk, an dem Halteteil angeordnet sein. Das Verstellelement des Halteteils kann wie zuvor beschrieben als Klappmechanismus und/oder als Schiebemechanismus ausgeführt sein.

Bei der Patientenaufnahme kann es sich beispielsweise um ein Sitzmöbel oder ein Liegemöbel handeln. Beispielsweise handelt es sich bei der Patientenaufnahme um einen Operationstisch, ein Bett, eine Krankenbahre oder ein beliebiges anderes Möbelstück, auf das der Patient zur Behandlung gebettet werden kann. Dadurch, dass der Patient ruhig auf der Patientenaufnahme verweilt, kann mittels des Halteteils, die Relativposition zwischen Patient und Nadelführung eingestellt werden. Mit anderen Worten ist das Halteteil zur Einstellung der Relativposition zwischen Nadelführung und Patienten ausgebildet, wenn die Führungsanordnung an der Patientenaufnahme angeordnet und der Patient auf der Patientenaufnahme angeordnet sind. Das Einstellen beziehungsweise Verstellen der Relativposition zwischen Befestigungsteil und Nadelführung erfolgt insbesondere anhand der Pose zwischen Ausrichtelement und der vorbestimmten Lichtverteilung. Eine solche vorbestimmte Lichtverteilung kann in Richtung der Patientenaufnahme projiziert werden, insbesondere wenn der Patient auf der Patientenaufnahme angeordnet ist.

Weiterhin gehört zur Erfindung eine Behandlungsanordnung zur Längsführung einer medizinischen Nadel, mit
- einer Patientenaufnahme zum Aufnehmen eines Patienten,
- der oben genannten Führungsanordnung, welche mittels des Befestigungsteils an der Patientenaufnahme angeordnet ist, und
- einer Lichtquelle zum Erzeugen der vorbestimmten Lichtverteilung entsprechend einem zuvor bestimmten Nadelpfad, entlang welchem die medizinische Nadel geführt werden soll, relativ zu der Patientenaufnahme, wobei
- die Nadelführung mittels des Halteteils in der vorgegebenen Pose festlegbar ist, wobei
- nur in der vorgegebenen Pose anhand der Lichtverteilung ein vorbestimmtes Lichtmuster durch das an der Nadelführung angeordnete Ausrichtelement erzeugbar ist, und wobei
- die Nadelführung dazu ausgebildet, in der vorgegebenen Pose die medizinische Nadel längs des zuvor bestimmten Nadelpfads zu führen. Insbesondere weist die Lichtquelle eine vorbestimmte Relativposition relativ zu der Patientenaufnahme und somit auch relativ zu der Führungsanordnung auf. Auf diese Weise kann sichergestellt werden, dass die vorbestimmte Lichtverteilung mittels der Lichtquelle auf bestimmungsgemäße Weise, nämlich entsprechend dem zuvor bestimmten Nadelpfad, auf die Patientenaufnahme, und somit auch auf den Patienten, wenn dieser auf der Patientenaufnahme angeordnet ist, projiziert wird.

Außerdem gehört zur vorliegenden Erfindung ein Verfahren zum Ausrichten einer Nadelführung, welche zur Längsführung einer medizinischen Nadel eingerichtet ist, mit folgenden Schritten:
- Erzeugen einer Lichtverteilung anhand eines zuvor bestimmten Nadelpfads, entlang welchem die medizinische Nadel geführt werden soll, und
- Ausrichten der Nadelführung für die medizinische Nadel in eine vorgegebene Pose relativ zu der Lichtverteilung, wobei nur in der vorgegebenen Pose anhand der Lichtverteilung ein vorbestimmtes Lichtmuster durch ein an der Nadelführung angeordnetes Ausrichtelement erzeugt wird.

In einem optionalen zusätzlichen Verfahrensschritt kann der Nadelpfad bestimmt werden. Beispielsweise kann der Nadelpfad im Rahmen einer Röntgenuntersuchung bestimmt werden. Beispielsweise wird im Rahmen der Röntgenuntersuchung eine bestimmte Stelle oder ein bestimmtes Organ (beziehungsweise dessen Repräsentation in der Simulation) eines Patienten oder Dummys zum Simulieren des Patienten gesucht. Der Nadelpfad kann dann an Knochen vorbei hin zu der Stelle oder dem Organ berechnet werden.

Der Verfahrensschritt des Ausrichtens der Nadelführung kann beispielsweise automatisiert durch zumindest einen Aktor an einem Halteteil beziehungsweise an einem Verstellelement einer oben beschriebenen Führungsanordnung vorgenommen werden. Alternativ kann der Schritt durch einen Roboter oder eine Person, insbesondere einen Arzt oder dessen Gehilfen, durchgeführt werden. Ausdrücklich nicht Teil des beanspruchten Verfahrens ist das Einführen einer medizinischen Nadel in den Körper eines Patienten beziehungsweise das Behandeln des Patienten mittels einer solchen medizinischen Nadel. Das Einführen der Nadel in einen Dummy zum Simulieren des Patienten durch eine auf die vorliegende Art und Weise ausgerichtete Nadelführung kann hingegen als Teil der Erfindung betrachtet werden.

Beispielsweise wird zum Ausrichten der Nadelführung zunächst das Ausrichtelement an der Nadelführung angeordnet beziehungsweise auf die Nadelführung aufgesteckt. Nach dem Ausrichten der Nadelführung kann das Ausrichtelement wiederum entfernt werden, um eine Längsführung der Nadelführung für ein Führen der Nadel freizugeben.

Eine Weiterbildung des Verfahrens sieht vor, dass die Lichtverteilung derart erzeugt wird, dass zwei Ebenen im Raum aufgespannt werden und der Nadelpfad parallel, insbesondere partiell identisch, zu einer Schnittgeraden der zwei Ebenen verläuft. Insbesondere wird die Lichtverteilung durch eine Lichtquelle einer oben beschriebenen Behandlungsanordnung erzeugt. Im Rahmen der Weiterbildung kann dann vorgesehen sein, dass der Nadelpfad mittels der Schnittgeraden der zwei Ebenen, welche durch die Lichtverteilung aufgespannt werden, visualisiert beziehungsweise dargestellt wird. Durch die vorbestimmte Lichtverteilung beziehungsweise durch die durch sie aufgespannten Ebenen können zwei sich schneidende Linien auf eine oben beschriebene Patientenaufnahme beziehungsweise auf den Patienten projiziert werden. Der Schnittpunkt der beiden Linien kann dabei eine Einstichstelle repräsentieren. Merkmale und Eigenschaften sowie Weiterbildungen, welche im Kontext eines Aspekts der Erfindung beschrieben beziehungsweise offenbart wurden, gelten analog für sämtliche beanspruchten Gegenstände. Beispielsweise gelten Merkmale die in Bezug auf das Ausrichtelement, die Nadelführung und die Ausrichtanordnung offenbart wurden, analog auch für die Führungsanordnung, die Behandlungsanordnung und das Verfahren. Das Verfahren wird insbesondere mit einer oben beschriebenen Ausrichtanordnung, einem oben beschriebenen Ausrichtelement, einer oben beschriebenen Führungsanordnung oder einer oben beschriebenen Behandlungsanordnung durchgeführt. Dementsprechend beziehen sich die einzelnen Aspekte der Erfindung jeweils aufeinander.

Im Folgenden wird die Erfindung anhand von Zeichnungen genauer erläutert. Hierzu zeigen:
- FIG 1: eine schematische Perspektivansicht eines erfindungsgemäßen Ausrichtelements in einer Nadelführung;
- FIG 2: eine weitere schematische Perspektivansicht des Ausrichtelements in der Nadelführung;
- FIG 3: das Einführen einer medizinischen Nadel in die Nadelführung; und
- FIG 4: eine Behandlungsanordnung mit der Nadelführung und dem Ausrichtelement.

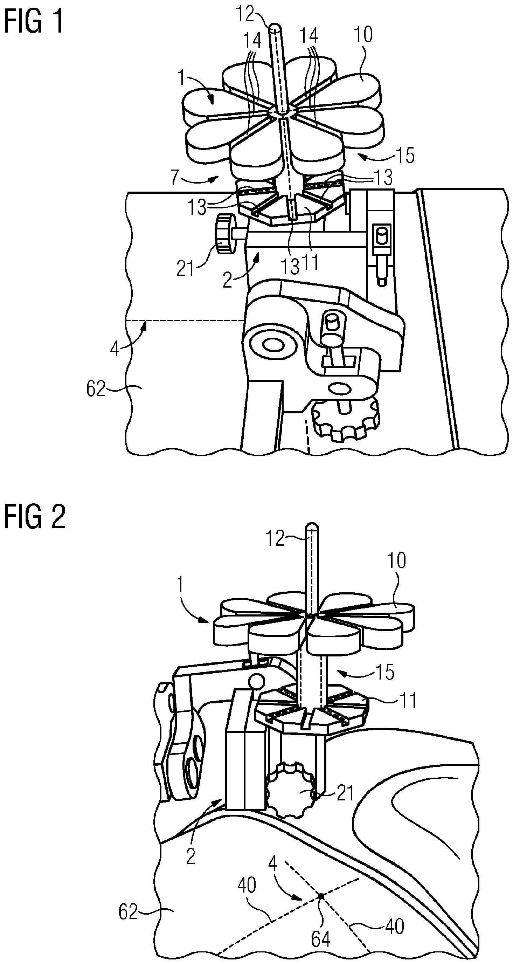

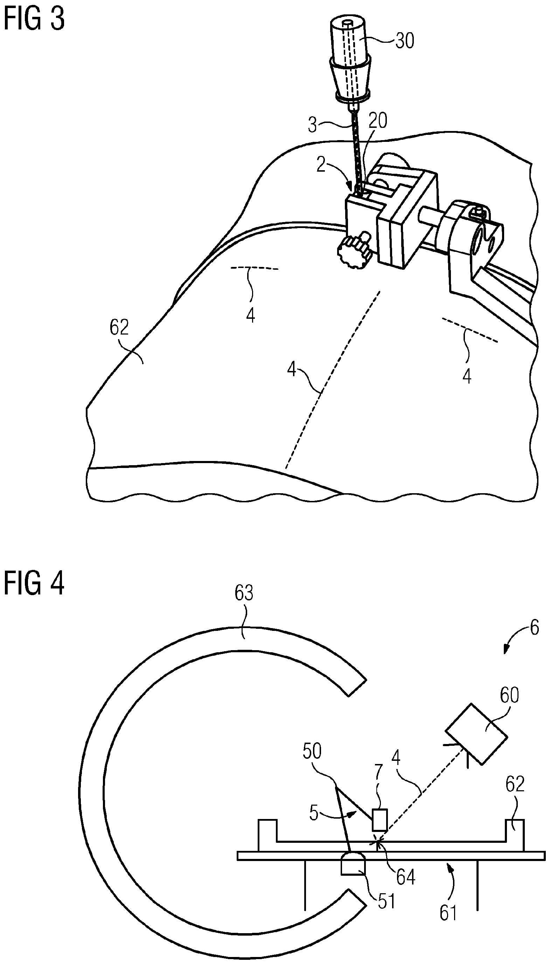

In FIG 1 ist ein Ausrichtelement 1 sowie eine Nadelführung 2 dargestellt. Zusammengefasst bilden das Ausrichtelement 1 und die Nadelführung 2 eine Ausrichtanordnung 7. Das Ausrichtelement 1 ist gemäß FIG 1 an einer Längsführung 20 der Nadelführung 2 angeordnet. Die Längsführung 20 ist in FIG 3 angedeutet und im vorliegenden Ausführungsbeispiel als Bohrung ausgeführt. Diese Bohrung ist insbesondere entlang einer Führungsrichtung der Längsführung 20 lang gestreckt. Lang gestreckt bedeutet in diesem Kontext, dass die Tiefe der Bohrung größer ist als ein Durchmesser der Bohrung. Insbesondere ist die Tiefe doppelt so groß oder fünfmal so groß wie der Durchmesser der Bohrung. Insbesondere geht die Bohrung durch die gesamte Nadelführung 2 hindurch. Mit anderen Worten kann eine medizinische Nadel 3 durch die Bohrung hindurch geführt werden. Auf diese Weise ist die Längsführung 20 der medizinischen Nadel durch die Nadelführung 2 bereitgestellt. Mit anderen Worten ist durch die Längsführung 20 eine Bewegung der Nadel 3 entlang zweier Raumrichtungen eingeschränkt beziehungsweise nicht möglich. Im vorliegenden Beispiel ermöglicht die Längsführung 20 somit eine Bewegung der Nadel 3 ausschließlich parallel zu der Führungsrichtung der Längsführung 20.

Nun wird wieder auf FIG 1 und FIG 2 Bezug genommen. Das Ausrichtelement 1 ist mittels eines als Stift ausgebildeten Verbindungselements an der Nadelführung 2 angeordnet. Dieser Stift des Verbindungselements ist ähnlich einem weiteren Stift 12 ausgebildet. Der Stift des Verbindungselements ist in Bezug auf den Stift 12 auf der gegenüberliegenden Seite des Ausrichtelements 1 angeordnet. Der Stift des Verbindungselements verläuft insbesondere in paralleler Verlängerung zu dem Stift 12. In den FIG 1 und FIG 2 ist das Verbindungselement sowie dessen Stift nicht sichtbar, da es sich innerhalb der Nadelführung 2 befindet. Insbesondere ist das Verbindungselement beziehungsweise dessen Stift innerhalb der Längsführung 20 beziehungsweise der Bohrung der Nadelführung 2 angeordnet. Mit anderen Worten ermöglicht der Stift ein Aufstecken des Ausrichtelements 1 auf die Nadelführung 2. Bei einem solchen Aufstecken wird der Stift in die Bohrung eingeführt. Optionalerweise umfasst die Nadelführung 2 eine Arretierung 21 zum Arretierung des Ausrichtelements 1 an der Nadelführung 2. Vorliegend ist die Arretierung 21 als Schraube ausgeführt.

Die FIG 4 zeigt eine Behandlungsanordnung 6, welche eine Führungsanordnung 5, eine Lichtquelle 60 und eine Patientenaufnahme 61 umfasst. Die Führungsanordnung 5 wiederum umfasst die Ausrichtanordnung 7, ein Befestigungsteil 51 sowie ein Halteteil 50. Die Lichtquelle 60 ist beispielsweise als Laser-Lichtquelle oder LED-Lichtquelle ausgeführt. Bei der Patientenaufnahme 61 kann es sich um einen OP-Tisch, ein Bett, eine Behandlungsliege oder jedes geeignete Sitzmöbel oder Liegemöbel handeln. Vorliegend umfasst die Behandlungsanordnung 6 außerdem ein Röntgengerät 63, insbesondere einen Röntgen-C-Bogen. Mittels des Röntgengeräts 63 kann eine Behandlung, im Rahmen welcher die medizinische Nadel 3 in einen Patienten eingeführt wird, überwacht werden. Hierzu kann das Röntgengerät 63 eine Fluoroskopie durchführen.

Mittels des Befestigungsteils 51 ist die Führungsanordnung 5 im vorliegenden Ausführungsbeispiel an der Patientenaufnahme 61 angeordnet. Dies erfolgt beispielsweise mittels eines Schraub-, eines Klemm-, oder eines beliebigen anderen Festlegemechanismus des Befestigungsteils 51. Das Halteteil 50 verbindet die Ausrichtanordnung 7 beziehungsweise die Nadelführung 2 mit dem Befestigungsteil 51 und im Falle dessen bestimmungsgemäße Befestigung an der Patientenaufnahme 61 auch mit der Patientenaufnahme 61.

Die Nadelführung 2 ist mittels des Halteteils 50 und des Befestigungsteils 51 relativ zu der Patientenaufnahme 61 festgelegt war, wobei mittels des Halteteils 50 ein Verstellen der Relativposition zwischen Nadelführung 2 und Patientenaufnahme 61 ermöglicht ist. Das Halteteil 50 kann ein Verstellelement zum Verstellen der genannten Relativposition aufweisen. Insbesondere können solche Verstellelemente Schienenmechanismus, Klappmechanismus oder Teleskopmechanismus ausgeführt sein. Insbesondere weist das Halteteil 50 mehrere verschiedenartige Verstellelemente auf. Auf diese Weise ermöglicht die Führungsanordnung 5 beziehungsweise Behandlungsanordnung 6 das Festlegen der Nadelführung 2 in einer vorbestimmten Position beziehungsweise Pose relativ zu einem auf der Patientenaufnahme 61 angeordneten Behandlungsobjekt 62. In weiterer Ausgestaltung kann das Halteteil 50 einen Motor (beispielsweise Elektromotor) beziehungsweise Aktoren zum automatisierten Verstellen der Relativposition aufweisen. Bei dem Behandlungsobjekt 62 kann sich beispielsweise um einen Patienten oder um einen Dummy zum Simulieren eines Patienten handeln.

Die Behandlungsanordnung 6 ermöglicht eine Behandlung eines Patienten mit der medizinischen Nadel 3 beziehungsweise eine Simulation einer solchen Behandlung anhand des Dummys. Zunächst kann eine Röntgenuntersuchung des Behandlungsobjekts 62 mittels des Röntgengeräts 63 vorgesehen sein. Im Rahmen dieser Röntgenuntersuchung kann ein Nadelpfad für die medizinische Nadel 3 bestimmt werden. Beispielsweise wird im Rahmen der Röntgenuntersuchung eine bestimmte Stelle oder ein bestimmtes Organ (beziehungsweise dessen Repräsentation in der Simulation) des Behandlungsobjekt 62 gesucht. Der Nadelpfad kann dann an Knochen vorbei hin zu der Stelle oder dem Organ berechnet werden. Anschließend wird mittels der Lichtquelle 60 entsprechend dem zuvor bestimmten Nadelpfad eine Lichtverteilung 4 abgestrahlt. Durch die Lichtverteilung 4 wird der Nadelpfad für die Nadel 3 visualisiert. Die Lichtverteilung 4 ist auch in FIG 2 zu erkennen. Durch die Lichtverteilung 4 wird eine Projektion 40 auf das Behandlungsobjekt 62 projiziert. Die Projektion 40 ist in FIG 2 in Form zweier senkrechter Linien dargestellt. Der Schnittpunkt der senkrechten Linien der Projektion 40 entspricht einer vorgesehenen Einstichstelle 64 für die Nadel 3. Durch die Lichtverteilung 4 werden dementsprechend zwei Ebenen im Raum aufgespannt. Hierbei können die Ebenen die Projektion 40 mit der Lichtquelle 60 verbinden. Die Projektion 40 kann somit als eine Projektion der beiden aufgespannten Ebenen parallel zur Lichtverteilung 4 auf die Oberfläche des Behandlungsobjekts 62 aufgefasst werden.

Anschließend erfolgt ein Ausrichten der Nadelführung 2 anhand der Lichtverteilung 4 mithilfe des Ausrichtelements 1. Wiederum in FIG 1 und FIG 2 zu sehen ist eine Lichtführungseinrichtung 15 des Ausrichtelements 1. Die Lichtführungseinrichtung 15 ist derart ausgeführt, dass diese nur bei einer durch deren Geometrie vorgegebenen Pose relativ zu der Lichtverteilung 4 ein vorbestimmtes Lichtmuster erzeugt. Hierzu weist die Lichtführungseinrichtung 15 zwei scheibenförmige Gebilde 10 und 11 auf. Die beiden scheibenförmige Gebilde 10, 11 sind in unterschiedlichen Ebenen angeordnet. Insbesondere verlaufen beide scheibenförmige Gebilde 10, 11 beziehungsweise die Ebenen, in welchen die scheibenförmige Gebilde 10, 11 angeordnet sind, jeweils senkrecht zu dem Stift 12 beziehungsweise zu dem Stift des Verbindungselements. Dementsprechend verlaufen die scheibenförmige Gebilde 10, 11 senkrecht zur Führungsrichtung der Längsführung 20, wenn das Ausrichtelement 1 bestimmungsgemäß an der Nadelführung 2 angeordnet ist.

Im vorliegenden Ausführungsbeispiel weist das scheibenförmige Gebilde 10 Spalte 12 auf. Die Spalte 12 dienen als Erkennungsmarken der entsprechenden Ebene. Das scheibenförmige Gebilde 11 weist Markierungen 13 auf. Die jeweiligen Markierungen 13 korrespondieren jeweils mit einem der Spalte 12. Insbesondere ist ein jeweiliger Spalt 12 in Führungsrichtung oberhalb der korrespondierenden Markierung 13 angeordnet. Vorliegend weist das Ausrichtelement 1 zwei Lichtführungseinrichtungen 15 auf. Jede der Lichtführungseinrichtungen 15 weist im vorliegenden Ausführungsbeispiel jeweils vier Spalte 12 und vier Markierungen 13 auf. Das vorliegende Ausrichtelement 1 weist somit insgesamt acht Spalte 12 und acht Markierungen 13 auf (siehe Bezugszeichen). Die Lichtführungseinrichtungen 15 sind zueinander um 45° gedreht.

Befindet sich die Nadelführung 2 nun in einer vorbestimmten Pose relativ zur Lichtverteilung 4 so tritt die Lichtverteilung 4 durch die Spalte 12 hindurch auf die Markierungen 13. Innerhalb der Behandlungsanordnung 6 befindet sich die Lichtquelle 60 und somit die Lichtverteilung 4 in einer definierten Relativposition zur Patientenaufnahme 61. Nur in der vorbestimmten Pose relativ zur Lichtverteilung 4 werden somit alle Markierungen 13 einer Lichtführungseinrichtung 15 durch die korrespondierenden Spalte 12 hindurch durch die Lichtverteilung 4 beleuchtet. Dies entspricht einem vorbestimmten Lichtmuster, welches durch die entsprechende Lichtführungseinrichtung 15 erzeugt wird. Das Lichtmuster ist in FIG 1 und FIG 2 durch gestrichelte Linien am Ausrichtelement 1 repräsentiert. Auf diese Weise kann die Nadelführung 2 mithilfe des Ausrichtelements 1 und mithilfe der Lichtverteilung 4 auf den zuvor bestimmten Nadelpfad ausgerichtet werden. Beispielsweise wird die Nadelführung 2 durch Verstellen des Halteteils 50 entsprechend der Lichtverteilung 4 ausgerichtet. Dieses Ausrichten kann beispielsweise durch den Motor oder die Aktoren des Halteteils 50, durch einen separaten Roboter oder durch eine Person, insbesondere ein Arzt oder dessen Gehilfen, durchgeführt werden.

Anschließend kann, wie in FIG 3 dargestellt, das Ausrichtelement 1 entfernt werden und die medizinische Nadel 3 durch die Längsführung 20 geführt werden. Das Einführen der Nadel 3 in das Behandlungsobjekt 62 kann optional mittels des Röntgengeräts 63 überwacht werden. Das Einführen der Nadel 3 in einen Patienten ist ausdrücklich nicht Teil der hier beanspruchten Erfindung. Das Einführen der Nadel 3 in einen Dummy zum Simulieren des Patienten durch eine auf die vorliegende Art und Weise ausgerichtete Nadelführung 2 kann hingegen als Teil der Erfindung betrachtet werden.

In der vorliegenden Ausführungsform handelt es sich beim Ausrichtelement 1 um ein aufsteckbares Ausrichtelement 1. Dieses kann aufgrund dessen Art und Form im vorliegenden Fall auch als "Aufsteckblume" bezeichnet werden. Hierbei entspricht Vorteilhafterweise die Ausrichtung des Ausrichtelements 1, insbesondere des Verbindungselements und/oder des Stifts 12, der Ausrichtung der Längsführung 20 beziehungsweise des durch die Längsführung 20 vorgegebenen Nadelpfades. Entsprechende Geraden, die durch die Längsführung 20 und das Ausrichtelement 1 definiert werden, sind dementsprechend parallel und insbesondere identisch. In anderen Ausführungsformen handelt kann das Ausrichtelement 1 fest mit der Nadelführung 2 verbunden sein. In diesem Fall kann das Ausrichtelement 1 insbesondere Teil der Nadelführung 2 sein und/oder einstückig mit dieser verbunden sein. In diesem Fall befindet sich das Ausrichtelement insbesondere nicht in dem durch die Längsführung 20 vorgegebenen Nadelpfad. In diesem Fall kann das Ausrichtelement 1 um eine feste Relativposition von der Längsführung 20 beziehungsweise dem durch die Einführung 20 vorgegebenen Nadelpfad verschoben sein. Aus diesem Grund kann es erforderlich sein, die Lichtverteilung 4 um dieselbe feste Relativposition zu verschieben. Hierdurch kann der korrekte Nadelpfad entsprechend dem zuvor bestimmten Nadelpfad auch in diesem Fall gewährleistet sein.

Insgesamt ist durch das Ausführungsbeispiel gezeigt, wie eine Genauigkeit beim Ausrichten einer Nadelführung verbessert werden kann. Außerdem kann eine verbesserte Genauigkeit beim Einführen der medizinischen Nadel ermöglicht werden. Durch die optimal ausgerichtete Nadelführung kann einerseits fehlerhafter Positionierung der Nadel und andererseits einem Zittern des Arztes entgegengewirkt werden.

## Patentansprüche

1. Ausrichtelement (1) zum Ausrichten einer Nadelführung (2), welche zur Längsführung einer medizinischen Nadel (3) eingerichtet ist, mit
- einem Verbindungselement zum Anordnen des Ausrichtelements (1) an einer solchen Nadelführung (2), und
- einer Lichtführungseinrichtung (15) für eine vorbestimmte Lichtverteilung (4), wobei die Lichtführungseinrichtung (15) nur bei einer durch deren Geometrie vorgegebenen Pose relativ zu der Lichtverteilung (4) ein vorbestimmtes Lichtmuster erzeugt,
**dadurch gekennzeichnet, dass**
- die Lichtführungseinrichtung (15) zumindest zwei Erkennungsmarken (13, 14) auf zwei unterschiedlichen Ebenen (10, 11) aufweist und die Erkennungsmarken (13, 14) nur im Falle der vorgegebenen Pose des Ausrichtelements (1) durch die vorbestimmte Lichtverteilung (4) gleichzeitig beleuchtet sind.

2. Ausrichtelement (1) nach Anspruch 1,
wobei das Verbindungselement zumindest partiell als Stift (12) ausgeführt ist.

3. Ausrichtelement (1) nach einem der Ansprüche 1 oder 2,
wobei die zwei Ebenen parallel sind.

4. Ausrichtelement (1) nach einem der Ansprüche 1 bis 3,
wobei dass die Lichtführungseinrichtung (15) ein erstes (10) und ein zweites (11) scheibenförmiges Gebilde aufweist, wobei das erste (10) und das zweite (11) scheibenförmige Gebilde jeweils in einer unterschiedlichen der Ebenen angeordnet sind, und wobei Erkennungsmarken der ersten Ebene als Spalte (14) und Erkennungsmarken der zweiten Ebene als hierzu korrespondierende Markierungen (13) ausgeführt sind.

5. Ausrichtanordnung (7), welche zur Längsführung einer medizinischen Nadel (3) eingerichtet ist, mit
- einer ausrichtbaren Nadelführung (2), und
- einem Ausrichtelement (1) nach einem der vorhergehenden Ansprüche.

6. Ausrichtanordnung (7) nach Anspruch 5, wobei die Nadelführung (2) zur Aufnahme des Ausrichtelements (1) und zur Führung der medizinischen Nadel (3) dasselbe Mittel aufweist.

7. Ausrichtanordnung (7) nach Anspruch 5, wobei dass die Nadelführung (2) und das Ausrichtelement (1) einstückig miteinander verbunden sind.

8. Ausrichtanordnung (7) nach einem der Ansprüche 5 bis 7, wobei zumindest eine der zwei unterschiedlichen Ebenen (10, 11) senkrecht zu einer Führungsrichtung der Nadelführung (2) ausgerichtet ist.

9. Ausrichtanordnung (7) nach einem der Ansprüche 5 bis 8, wobei sich eine Projektionen des ersten (10) und des zweiten (11) scheibenförmigen Gebildes in Richtung der Längsführung (20) auf eine der Ebenen zumindest bereichsweise überschneiden.

10. Führungsanordnung (5) zur Längsführung einer medizinischen Nadel (3), mit
- einer Ausrichtanordnung (7) nach einem der Ansprüche 5 bis 9,
- einem Befestigungsteil (51) zum Befestigen der Führungsanordnung an einer Patientenaufnahme zur Aufnahme eines Patienten, und
- einem Halteteil (50) zum Verbinden der Nadelführung (2) und des Befestigungsteils (51) miteinander, wobei der Halteteil (50) zumindest ein Verstellelement zum Verstellen einer Relativposition zwischen Befestigungsteil (51) und Nadelführung (2) aufweist.

11. Behandlungsanordnung (6) zur Längsführung einer medizinischen Nadel (3), mit
- einer Patientenaufnahme (61) zum Aufnehmen eines Patienten,
- einer Führungsanordnung (5) nach Anspruch 10, welche mittels des Befestigungsteils (51) an der Patientenaufnahme (61) angeordnet ist, und
- einer Lichtquelle (60) zum Erzeugen der vorbestimmten Lichtverteilung (4) entsprechend einem zuvor bestimmten Nadelpfad, entlang welchem die medizinische Nadel (3) geführt werden soll, relativ zu der Patientenaufnahme (61), wobei
- die Nadelführung (2) mittels des Halteteils (50) in der vorgegebenen Pose festlegbar ist, wobei
- nur in der vorgegebenen Pose anhand der Lichtverteilung (4) ein vorbestimmtes Lichtmuster durch das an der Nadelführung (2) angeordnete Ausrichtelement (1) erzeugbar ist, und wobei
- die Nadelführung (2) dazu ausgebildet, in der vorgegebenen Pose die medizinische Nadel (3) längs des zuvor bestimmten Nadelpfads zu führen.

12. Verfahren zum Ausrichten einer Nadelführung (2), welche zur Längsführung einer medizinischen Nadel (3) eingerichtet ist, mit den Schritten:
- Erzeugen einer Lichtverteilung (4) anhand eines zuvor bestimmten Nadelpfads, entlang welchem die medizinische Nadel (4) geführt werden soll, und
- Ausrichten der Nadelführung (2) für die medizinische Nadel in eine vorgegebene Pose relativ zu der Lichtverteilung (4), wobei nur in der vorgegebenen Pose anhand der Lichtverteilung (4) ein vorbestimmtes Lichtmuster durch ein an der Nadelführung (2) angeordnetes Ausrichtelement (1) erzeugt wird, wobei zumindest zwei Erkennungsmarken (13, 14) auf zwei unterschiedlichen Ebenen (10, 11) vorgesehen werden und die Erkennungsmarken (13, 14) nur im Falle der vorgegebenen Pose durch die vorbestimmte Lichtverteilung (4) gleichzeitig beleuchtet werden.

13. Verfahren nach Anspruch 12, wobei
die Lichtverteilung (4) derart erzeugt wird, dass die zwei Ebenen im Raum aufgespannt werden und der Nadelpfad parallel, insbesondere partiell identisch, zu einer Schnittgeraden der zwei Ebenen verläuft.

## Claims

1. Aligning element (1) for aligning a needle guide (2), which is equipped for longitudinally guiding a medical needle (3), comprising
- a connecting element for arranging the aligning element (1) on such a needle guide (2), and
- a light-guiding apparatus (15) for predetermined diffusion of light (4), wherein the light-guiding apparatus (15) only generates a predetermined light pattern in a pose relative to the diffusion of light (4), which pose is predetermined by the geometry of said apparatus,
**characterised in that**
- the light-guiding apparatus (15) has at least two identifiers (13, 14) on two different planes (10, 11) and the identifiers (13, 14) are only illuminated simultaneously by the predetermined diffusion of light (4) in the case of the predetermined pose of the aligning element (1).

2. Aligning element (1) according to claim 1,
wherein the connecting element is at least partly embodied as a pin (12).

3. Aligning element (1) according to one of claims 1 or 2, wherein the two planes are parallel.

4. Aligning element (1) according to one of claims 1 to 3, wherein the light-guiding apparatus (15) has a first (10) and a second (11) disc-shaped structure, wherein the first (10) and the second (11) disc-shaped structure are each arranged in a different one of the planes, and wherein identifiers in the first plane are embodied as slits (14) and identifiers in the second plane as markings (13) corresponding therewith.

5. Aligning arrangement (7), which is equipped for longitudinally guiding a medical needle (3), comprising
- an adjustable needle guide (2), and
- an aligning element (1) according to one of the preceding claims.

6. Aligning arrangement (7) according to claim 5, wherein the needle guide (2) has the same means for accommodating the aligning element (1) and for guiding the medical needle (3).

7. Aligning arrangement (7) according to claim 5, wherein the needle guide (2) and the aligning element (1) are combined with each other in one piece.

8. Aligning arrangement (7) according to one of claims 5 to 7, wherein at least one of the two different planes (10, 11) is aligned perpendicular to a guide direction of the needle guide (2) .

9. Aligning arrangement (7) according to one of claims 5 to 8, wherein projections of the first (10) and of the second (11) disc-shaped structure overlap in the direction of the longitudinal guide (20) onto one of the planes at least in some areas.

10. Guiding arrangement (5) for longitudinally guiding a medical needle (3), comprising
- an aligning arrangement (7) according to one of claims 5 to 9,
- a fixing component (51) for affixing the guiding arrangement onto a patient-accommodating unit for receiving a patient, and
- a holder (50) to combine the needle guide (2) and the fixing component (51) with each other, wherein the holder (50) has at least one adjustment element for adjusting a relative position between the fixing component (51) and the needle guide (2).

11. Treatment arrangement (6) for longitudinally guiding a medical needle (3), comprising
- a patient-accommodating unit (61) for receiving a patient,
- a guiding arrangement (5) according to claim 10, which is arranged on the patient-accommodating unit (61) by means of the fixing component (51) and
- a light source (60) for generating the predetermined diffusion of light (4) according to a previously determined needle pathway along which the medical needle (3) is to be guided relative to the patient-accommodating unit (61), wherein
- the needle guide (2) can be fixed in the predetermined pose by means of the holder (50), wherein
- it is only in the predetermined pose that a predetermined light pattern can be generated by means of the diffusion of light (4) by the aligning element (1) that is arranged on the needle guide (2), and wherein
- the needle guide (2) is embodied to guide the medical needle (3) in the predetermined pose along the previously determined needle pathway.

12. Method for aligning a needle guide (2), which is equipped for longitudinally guiding a medical needle (3), comprising the steps:
- generating a diffusion of light (4) by means of a previously determined needle pathway, along which the medical needle (4) is to be guided, and
- aligning the needle guide (2) for the medical needle into a predetermined pose relative to the diffusion of light (4), wherein it is only in the predetermined pose by means of the diffusion of light (4) that a predetermined light pattern is generated by an aligning element (1) that is arranged on the needle guide (2), wherein at least two identifiers (13, 14) on two different planes (10, 11) are provided and the identifiers (13, 14) are only illuminated simultaneously by the predetermined diffusion of light (4) in the case of the predetermined pose.

13. Method according to claim 12, wherein the diffusion of light (4) is generated such that two spatial planes are spanned and the needle pathway runs parallel, in particular partly identically, to a line of intersection of the two planes.

## Revendications

1. Elément (1) d'alignement pour l'alignement d'un guide (2) d'aiguille, qui est conçu pour le guidage longitudinal d'une aiguille (3) médicale, comprenant
- un élément de liaison pour monter l'élément (1) d'alignement sur un guide (2) d'aiguille de ce genre, et
- un dispositif (15) de guidage de la lumière pour une répartition (4) définie à l'avance de la lumière, dans lequel le dispositif (15) de guidage de la lumière produit un motif de lumière défini à l'avance seulement pour une pose, donnée à l'avance par sa géométrie, par rapport à la répartition (4) de la lumière, **caractérisé en ce que**
- le dispositif (15) de guidage de la lumière a au moins deux repères (13, 14) de détection sur deux plans (10, 11) différents et les repères (13, 14) de détection ne sont éclairés simultanément, par la répartition (4) définie à l'avance de la lumière, que dans le cas de la pose donnée à l'avance de l'élément (1) d'alignement.

2. Elément (1) d'alignement suivant la revendication 1,
dans lequel l'élément de liaison est réalisé au moins en partie sous la forme d'une broche (12).

3. Elément (1) d'alignement suivant l'une des revendications 1 ou 2,
dans lequel les deux plans sont parallèles.

4. Elément (1) d'alignement suivant l'une des revendications 1 à 3,
dans lequel le dispositif (15) de guidage de la lumière a une première (10) et une deuxième (11) structures en forme de disque, la première (10) et la deuxième (11) structures en forme de disque étant disposées dans un différent des plans et dans lequel des repères de détection du premier plan sont réalisés sous la forme de fentes (14) et des repères de détection du deuxième plan sont réalisés sous la forme de repères (13) y correspondant.

5. Dispositif (7) d'alignement, qui est conçu pour le guidage longitudinal d'une aiguille (3) médicale, comprenant
- un guide (2) d'aiguille pouvant être alignée, et
- un élément (1) d'alignement suivant l'une des revendications précédentes.

6. Dispositif (7) d'alignement suivant la revendication 5,
dans lequel le guide (2) d'aiguille a le même moyen pour la réception de l'élément (1) d'alignement et pour le guidage de l'aiguille (3) médicale.

7. Dispositif (7) d'alignement suivant la revendication 5,
dans lequel le guide (2) d'aiguille et l'élément (1) d'alignement sont reliés l'un à l'autre en une seule pièce.

8. Dispositif (7) d'alignement suivant l'une des revendications 5 à 7,
dans lequel au moins l'un des deux plans (10, 11) différents est perpendiculaire à une direction de guidage du guide (2) d'aiguille.

9. Dispositif (7) d'alignement suivant l'une des revendications 5 à 8,
dans lequel une projection de la première (10) et de la deuxième (11) structure en forme de disque en direction du guidage (20) longitudinal sur l'un des plans se recouvrent au moins en partie.

10. Dispositif (5) de guidage pour le guidage longitudinal d'une aiguille (3) médicale, comprenant
- un dispositif (7) d'alignement suivant l'une des revendications 5 à 9,
- une partie (51) de fixation pour la fixation du dispositif de guidage à un enregistrement de patient pour l'enregistrement d'un patient, et
- une partie (50) de maintien pour relier le guide (2) d'aiguille et la partie (51) de fixation entre eux, la partie (50) de maintien ayant au moins un élément de réglage pour le réglage d'une position relative entre la partie (51) de fixation et le guide (2) d'aiguille.

11. Dispositif (6) de manipulation pour le guidage longitudinal d'une aiguille (3) médicale, comprenant
- un enregistrement (61) de patient pour l'enregistrement d'un patient,
- un dispositif (5) de guidage suivant la revendication 10, qui est monté sur l'enregistrement (61) du patient au moyen de la partie (51) de fixation, et
- une source (60) lumineuse de production de la répartition (4) définie à l'avance de la lumière correspondant à un trajet d'aiguille défini à l'avance, le long duquel l'aiguille (3) médicale doit être guidée, par rapport à l'enregistrement (61) de patient, dans lequel
- le guide (2) d'aiguille peut être fixé dans la pose donnée à l'avance au moyen de la partie (50) de maintien, dans lequel
- seulement dans la pose donnée à l'avance, il peut être produit, à l'aide de la répartition (4) de lumière, un motif de lumière défini à l'avance par l'élément (1) d'alignement monté sur le guide (2) d'aiguille, et dans lequel
- le guide (2) d'aiguille est constitué pour, dans la pose donnée à l'avance, guider l'aiguille (3) médicale le long du trajet d'aiguille défini à l'avance.

12. Procédé d'alignement d'un guide (2) d'aiguille, qui est conçu pour le guidage longitudinal d'une aiguille (3) médicale, comprenant les stades :
- production d'une répartition (4) de lumière à l'aide d'un trajet d'aiguille défini à l'avance, le long duquel l'aiguille (4) médicale doit être guidée, et
- alignement du guide (2) d'aiguille pour l'aiguille médicale dans une pose donnée à l'avance par rapport à la répartition (4) de la lumière, dans lequel on ne produit que dans la pose donnée à l'avance, à l'aide de la répartition (4) de la lumière, un motif de lumière défini à l'avance par un élément (1) d'alignement monté sur le guide (2) d'aiguille, dans lequel on prévoit au moins deux repères (13, 14) de détection sur deux plans (10, 11) différents et on éclaire en même temps, par la répartition (4) de lumière définie à l'avance, les repères (13, 14) de détection seulement dans le cas de la pose donnée à l'avance.

13. Procédé suivant la revendication 12,
dans lequel on produit la répartition (4) de la lumière de manière à ce que les deux plans se déploient dans l'espace et le trajet de l'aiguille s'étend parallèlement, en étant en partie identique, à une droite de coupe des deux plans.
